# EUROPEAN PATENT APPLICATION

(11) **EP 1 982 976 A1**
(43) Date of publication of application: **22.10.2008**
(21) Application number: 07090075.8
(22) Date of filing: 18.04.2007
(51) Int. Cl.: C07D 209/08, C07D 209/44, C07D 307/79, C07D 307/87, C07D 333/54, C07D 333/72, C07D 405/08

(54) **Process for the manufacture of non-steroidal anti-inflammatory agents and intermediates thereof**

(71) Applicant: Intendis GmbH, 10589 Berlin (DE)
(72) Inventor: Schweizer, Steffen, Dr., 12163 Berlin (DE)
(74) Representative: Seuss, Thomas

(57) **Abstract**

The current invention describes novel synthetic routes and intermediates for the manufacture of anti-inflammatory agents of general formula VIII 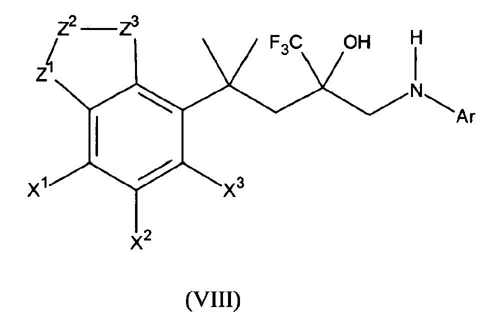 in which one of the groups X¹, X², X³ is selected from
fluoro, chloro, bromo, hydroxy, methoxy, ethoxy, trifluoromethyl, amino whereas the other groups X¹, X², X³ have the meaning of a hydrogen atom,
in which one of the groups Z¹, Z², Z³ is selected from
-O-, -S-, -NH-, -N(-CH₃)-,
whereas the other groups Z¹, Z², Z³ have the meaning of a -CH₂- group,
and in which Ar is an aromatic group.

## Description

### Background of the Invention

Compounds of general formula VIII 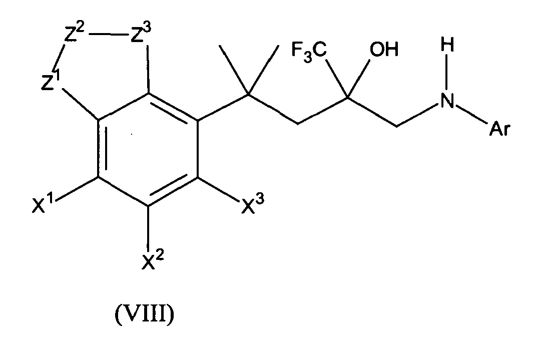 in which one of the groups X¹, X², X³ is selected from
fluoro, chloro, bromo, hydroxy, methoxy, ethoxy, trifluoromethyl, amino
whereas the other groups X¹, X², X³ have the meaning of a hydrogen atom,
in which one of the groups Z¹, Z², Z³ is selected from
-O-, -S-, -NH-, -N(-CH₃)-,
whereas the other groups Z¹, Z², Z³ have the meaning of a -CH₂- group,
and in which Ar is an aromatic group
are described as powerful anti-inflammatory agents (e.g. WO 98/54159, WO 00/32584, WO 02/10143, WO 03/082827, and WO 2006/050998).

However, the processes for the manufacturing of the compounds of general formula VIII have quite a number of steps, resulting in low yields of the whole chain of reactions and are not suitable for large scale productions.

### Object of the Invention

It is therefore the object of the invention to make available a novel process characterized in a lower number of steps with a higher total yield which is suitable for pharmaceutical production. The object of the invention is achieved by the processes described herein.

### General Description of the Invention and preferred Embodiments

An essential element of the synthetic route described herein is the compound of general formula I 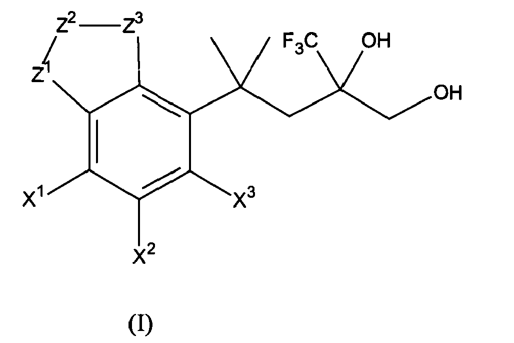 in which one of the groups X¹, X², X³ is selected from
fluoro, chloro, bromo, hydroxy, methoxy, ethoxy, trifluoromethyl, amino
whereas the other groups X¹, X², X³ have the meaning of a hydrogen atom,
and in which one of the groups Z¹, Z², Z³ is selected from
-O-, -S-, -NH-, -N(-CH₃)-,
whereas the other groups Z¹, Z², Z³ have the meaning of a-CH₂- group.

Another aspect of the invention is a manufacturing method according to which the compounds of general formula VIII can be produced in an enantiomerically pure form (enantiomeric excess ee > 80%). It is clear to the expert in the art that the compounds of the prior art are - when used as pharmaceuticals - usually in an enantiomerically pure form. It is therefore important to develop a manufacturing route that is able to produce the compounds of general formula VIII in enantiomerically pure form. This object is also achieved by the present invention. One of the starting materials of the process described herein (alkyl 2-hydroxy-4-methyl-2-(trifluoromethyl)pentenoate) may be used in the described processes in enantiomerically pure form, subsequently yielding the final coumpound in enantiomerically pure form.

The compound alkyl 2-hydroxy-4-methyl-2-(trifluoromethyl)pentenoate may be generated according to the method described by Mikami (Tetrahedron: Asymmetry 15 (2004) 3885-3889). It is also possible to use racemic alkyl 2-hydroxy-4-methyl-2-(trifluoromethyl)-pentenoate and separate the enantiomers by enzymatic hydrolysis.

Using enantiomerically pure alkyl 2-hydroxy-4-methyl-2-(trifluoromethyl)pentenoate as one of the starting materials results in an enantiomerically pure compound of general formula VIII. The advantage of the described reaction starting with enantiomerically pure alkyl 2-hydroxy-4-methyl-2-(trifluoromethyl)pentenoate is that it avoids the production of an unwanted enantiomer, therefore avoiding the separation of the enantiomers at a later stage (or even in the final product) and therefore being much more efficient.

The general method for the production of the compounds of general formula VIII via the compound of general formula I is described below in detail. The expert in the art is fully aware of the fact that a number of variants of the reaction route are possible without deviating from the general teaching of the present invention. It is for example possible to not isolate all intermediates of the synthetic route.

The process for the manufacturing starts with a compound of general formula IV 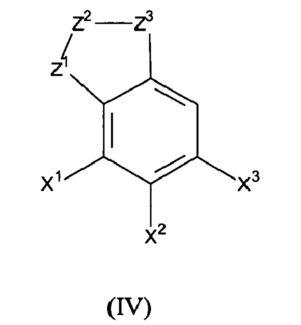 in which X¹, X¹, X¹, Z¹, Z², Z³ have meaning described above.

The compound of general formula N is reacted with alkyl 2-hydroxy-4-methyl-2-(trifluoromethyl)pentenoate to yield a compound of general formula III 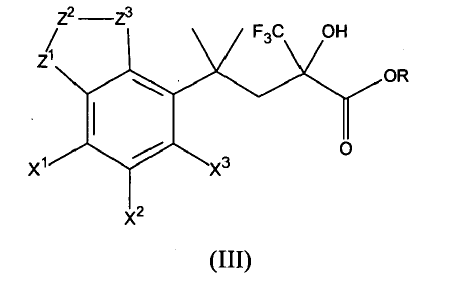 in which X¹, X², X³, Z¹, Z², Z³ have the above described meaning and in which R is a C₁-C₅-alkyl group, which might be straight or branched.

The alkyl group R in the alkyl 2-hydroxy-4-methyl-2-(trifluoromethyl)pentenoate 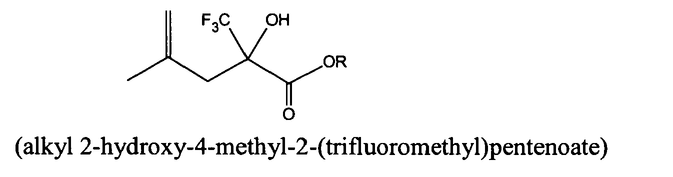 is a C₁-C₅-alkyl group which might be straight or branched, e.g. a methyl, ethyl, propyl, isopropyl, n-butyl, iso-butyl, or pentyl group. Preferably R is an ethyl group.

The reaction described above is carried out in a organic solvent in the presence of a lewis acid. Suitable solvents are e.g. polar solvents or halogenated solvents, the preferred solvents include dichloromethane and dichloroethane. The lewis acid may be aluminium chloride, BF₃, HF, or phosphoric acid.

In a preferred embodiment of the invention the compound according to formula IV is solved in a halogenated solvent (e.g. CH₂Cl₂) AlCl₃ is added and finally the alkyl 2-hydroxy-4-methyl-2-(trifluoromethyl)pentenoate is added to the stirred solution. In an even more preferred embodiment the addition of AlCl₃ and the alkyl 2-hydroxy-4-methyl-2-(trifluoromethyl)pentenoate is carried out at 0-5°C, the mixture is allowed to come to room temperature and the mixture is continued to stir for 1-5 days at room temperature.

It is furthermore preferred that 1,5 equivalents of the compound according to formula IV are used, 2 equivalents AlCl₃ and 1,0 equivalent of ethyl 2-hydroxy-4-methyl-2-(trifluoromethyl)pentenoate.

The reaction described above can be carried out with enantiomerically pure alkyl 2-hydroxy-4-methyl-2-(trifluoromethyl)pentenoate. The enantiomeric pure 2-hydr6xy-4-methyl-2-(trifluoromethyl)pentenoate may be synthesized under asymmetric catalysis as described by Mikami (see above) or the racemic form may be enzymatically hydrolized.

The asymmetric hydrolysis may be carried out in a buffered DMSO solution. Quite a number of enzymes are possible for the enzymatic hydrolysis. These include the hydrolases (EC3.hydrolases) of the subclasses EC3.1. (carboxylic esterhydrolasis in particular).

Such hydrolases are commercially available from various sources, e.g.
**1. Amano Enzyme Inc., Japan**
   Lipase AH, Lipase AK, Lipase AYS, Lipase PS, Protease K, Protease N, Protease P
**2. Alphamerics Limited, UK**
   Lipase C1, Lipase C2, Lipase A, Lipase AS1, Lipase AN, Lipase PC, Lipase PF, Lipase B (CALB)
**3. Biocatalytics Incorporated., USA**
   ICR-101, ICR-102, ICR-103, ICR-104, ICR-105, ICR-106, ICR-107, ICR-108, ICR-109, ICR-110, ICR-111, ICR-112, ICR-113, ICR-114, ICR-115, ICR-116, ICR-117, ICR-118, ICR-119, ICR-120, IMW-102, IMW-105
**4. Julich Chiral Solutions, Germany**
   Esterase BS1, Esterase BS2, Esterase BS3, Esterase PF2, Esterase PL
**5. NovoNordisk / Novozyme (Denmark)**
   Duramyl, Novozyme 868, Novozyme 525L, Novozyme 388, Neutrase 0, Liopoase
**6. Sigma, Germany**
   Lipase from porcine pancreas Typ II, Esterase porcine liver, Lipase candida rugosa. The enzymatic hydrolysis is carried out as follows: Racemic alkyl 2-hydroxy-4-methyl-2-(trifluoromethyl)pentenoate is used as starting material. It is emulsified in DMSO, the buffered enzyme is added at temperature from about 30°C to about 60°C. Temperature and reaction time depend from enzyme to enzyme. The reaction time may be up to 300 hours. The reaction conditions have to be tested under control (e.g. GC control) to find the optimum.

The compound of general formula III described above is then saponified to form a compound of general formula II 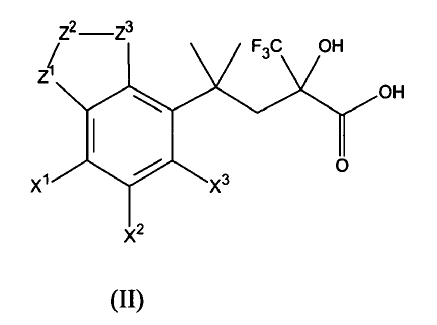 in which X¹, X², X³, Z¹, Z², Z³ have the meaning described above.

The saponification may be carried out under standard conditions, e.g. in ethanol with the use of sodium hydroxide. The step can be applied to the compound of general formula III directly from the previous step without isolation of the compound of general formula III.

The compound of general formula II may be reduced to the key compound of general formula I 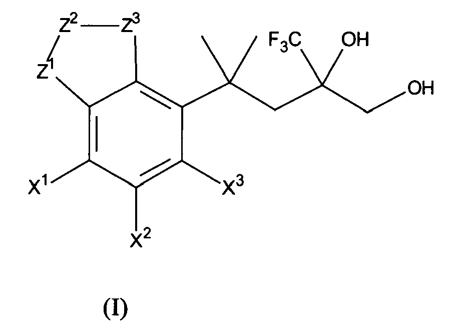 by e.g. lithium aluminium hydride or lithium boronhydride.

Enantiomerically pure compounds of general formula I are key compounds of the process, and are therefore a further object of the invention. Preferred embodiments of the compounds of formula I are those which have one of the following substitution patterns:

| | **Z¹** | **Z²** | **Z³** | **X¹** | **X²** | **X³** | **enantiomer** |
|---|---|---|---|---|---|---|---|
| a) | O | | | F | | | *R* |
| b) | | O | | | F | | *R* |
| c) | | | O | | | F | *R* |
| d) | NH | | | F | | | *R* |
| e) | | | O | | F | | *R* |
| f) | S | | | | | F | *R* |
| g) | | NH | | Cl | | | *R* |
| h) | | | NH | | Cl | | *R* |
| i) | | | S | | | Cl | *R* |
| j) | | S | | CF₃ | | | *R* |
| k) | S | | | | CF₃ | | *R* |
| l) | O | | | | | CF₃ | *R* |
| m) | | O | | O-CH₃ | | | *R* |
| n) | | | O | | O-CH₃ | | *R* |
| o) | | O | O | | | O-CH₃ | *R* |
| p) | O | | | | F | | *R* |
| q) | NH | | | | | F | *R* |
| r) | | NH | | NH₂ | | | *R* |
| s) | | | NH | | NH₂ | | *R* |
| t) | | | O | | | Br | *R* |
| u) | O | | | F | | | *S* |
| v) | | O | | | F | | *S* |
| w) | | | O | | | F | *S* |
| x) | NH | | | F | | | *S* |
| y) | | | O | | F | | *S* |
| z) | S | | | | | F | *S* |
| aa) | | NH | | Cl | | | *S* |
| bb) | | | NH | | C1 | | *S* |
| cc) | | | S | | | C1 | *S* |
| dd) | | S | | CF₃ | | | *S* |
| ee) | S | | | | CF₃ | | *S* |
| ff) | O | | | | | CF₃ | *S* |
| gg) | | O | | O-CH₃ | | | *S* |
| hh) | | | O | | O-CH₃ | | *S* |
| ii) | | O | O | | | O-CH₃ | *S* |
| jj) | O | | | | F | | *S* |
| kk) | NH | | | | | F | *S* |
| ll) | | NH | | NH₂ | | | *S* |
| mm) | | | NH | | NH₂ | | *S* |
| nn) | | | O | | | Br | *S* |

Enantiomerically pure in the context of this invention means an enantiomeric excess (ee) > 80%. It has to be understood that according to the present invention it is possible to synthesize compounds of ee > 90%, ee> 95% and even ee> 97%.

The compound of general formula I is then oxydized to form the aldehyde of general formula V 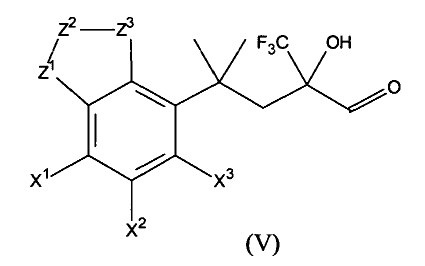 in which X¹, X², X¹, Z¹, Z², Z³ have the meaning described above.

The oxidation may be carried out by SO₃/pyridin complex or with oxalylchloride/DMSO (Swern oxidation). The expert in the art is aware of other possibilities to oxydize the alcohol of formula I to the aldehyd of formula V.

The aldehyde of general formula V is then reacted with an aromatic amine of general formula VI

H₂N-Ar (VI)

in which Ar is an aromatic group.

The compound according to general formula VI may be any aromatic amine. Preferred embodiments of the compounds of general formula VI are the compounds:
5-amino-2-methylquinoline
5-amino-quinoline
3-amino-quinoline
5-amino-1-methyl-isoquinoline
5- amino-isoquinoline
5-amino-isochinol-2(1H)-one
5-amino-2,6-di-methylquinoline
5-amino-6-chloro-2-methylquinoline
5-amino-6-fluoro-2-methylquinoline
5-amino-2-methyl-isochinol- (2H)-one
amino-benzene
1-amino-2-methyl-benzene
1-amino-4-methyl-benzene
2-amino-pyrimidine
4-amino-pyrimidine
2-amino-pyridine
4-amino-pyridine
2-amino-4-methylpyridine
N-(4-aminophenyl)-piperidine
5-amino-2-methyl-indole

The generated imine of formula VII 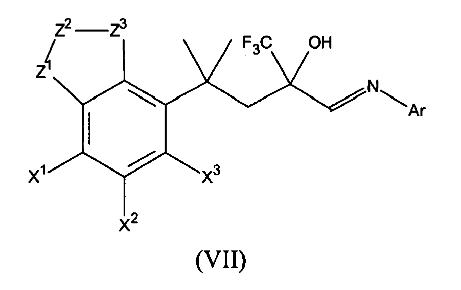 in which X¹, X², X³, Z¹, Z², Z³ and Ar have the meaning described above
is subsequently reduced in order to yield the compound according to general formula VIII. The reaction may be carried out by sodium boronhydride in alcoholic solution, it may also be carried out by H₂/Ni.

As described above the expert in the art knows a number of variations and deviations from the process steps described herein. It is therefore clear that the invention described in the claims encompasses further variants and deviations which are obvious to the expert in the art or can easily be identified by the expert in the art without any need to be inventive.

The process steps described above are furthermore described in the following examples which are not meant to limit the invention in any way.

### Examples

### 1) Synthesis of ethyl-2-hydroxy-4-methyl-2-(trifluoromethyl)pentenoate

A suspension of 0,27 mol Mn and 0,01 mol ZnCl₂ in 105 ml THF is heated to reflux. 0,01 mol 3-bromo-2-methyl-1-propene is added to the boiling mixture and after 30 minutes a solution of 0,11 mmol ethyl-trifluoropyruvate and 0,18 mol 2-bromo-2-methyl-1-propene in 90 ml THF is dropped to the reaction mixture within 2,5 hours. After 3 hours under reflux the mixture is stirred for 19 hours at room temperature. The reaction mixture is poured on 90 ml of a saturated NH₄Cl and ice mixture. After vigorous stirring for 30 minutes the mixture is extracted four times with 110 ml of MTBE each. The combined organic extracts are washed with 30 ml of brine, dried over magnesiumsulfate and concentrated in vacuo. The residue is destilled under reduced pressure. ethyl-2-hydroxy-4-methyl-2-(trifluoromethyl)pentenoate is obtained in 73% yield.

### 2) Synthesis of ethyl-4-(5-fluoro-2,3-dihydro-benzofuran-7-yl)-2-hydroxy-4-methyl-2-(trifluoromethyl)pentanoate

A solution of 0,07 mol 5-fluoro-2,3-dihydro-benzofurane in 21 ml of dichloromethan is cooled to 3°C. To this solution 0,1 mol of AlCl₃ is added over a period of 30 minutes. After this addition 0,05 mol of ethyl-2-hydroxy-4-methyl-2-(trifluoromethyl)pentenoate is added dropwise over 30 minutes. The mixture is stirred for 2-3 days at 25°C. After complete reaction the solution is poured on a mixture of ice (50 ml) and 1M HCl (10 ml) and stirred for at least 1 hour. The aqueous phase is extracted three times with 51 ml of ethylacetate. The combined organic phases are washed with water, saturated sodium chloride solution (brine) and dried over magnesiumsulfate. The solvent is evaporated under vacuum. The product is recrystalized from n-heptane (product yield 61,3 %).
The same reaction described above may be carried out with other compounds according to formula IV 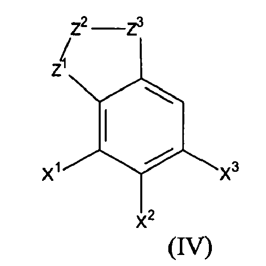 wherein X¹, X², X³, Z¹, Z², Z³ have the meaning according to the following table:

| | Z¹ | Z² | Z³ | X¹ | X² | X³ |
|---|---|---|---|---|---|---|
| a | O | | | F | | |
| b | | O | | | F | |
| c | | | O | | | F |
| d | NH | | | F | | |
| e | | | O | | F | |
| f | S | | | | | F |
| g | | NH | | Cl | | |
| h | | | NH | | Cl | |
| i | | | S | | | Cl |
| j | | S | | CF₃ | | |
| k | S | | | | CF₃ | |
| l | O | | | | | CF₃ |
| m | | O | | O-CH₃ | | |
| n | | | O | | O-CH₃ | |
| o | | O | O | | | O-CH₃ |
| p | O | | | | F | |
| q | NH | | | | | F |
| r | | NH | | NH₂ | | |
| s | | | NH | | NH₂ | |
| t | | | O | | | Br |

### 3) Synthesis of [4-(5-fluoro-2,3-dihydrobenzo-furan-7-yl)-4-methyl-2-(trifluoromethyl)pentane-1,2-diol]

A solution of 6,6 mol ethyl-4-(5-fluoro-2,3-dihydro-benzofuran-7-yl)-2-hydroxy-4-methyl-2-(trifluoromethyl)pentanoate in 77 ml of MTBE is cooled to 4°C. 12 mmol of lithium aluminiumhydride is added portionwise to the solution. The mixture is stirred at 4°C for 60 minutes, warmed to room temperature and stirred for at least 16 hours. After complete reaction (TLC control) the mixture is cooled to 4°C and treated with 1 ml of saturated (NH₄)₂CO₃ solution. The mixture is stirred for at least 2 hours whereupon the colour of the mixture turns from grey to white. The precipitated aluminium salts are filtered off and washed with 10 ml of MTBE. The solvent is evaporated under vacuum. The residue is purified by recrystalization from dichloromethane and N-heptane. (yield 73,7%).

Using the compounds according to the table in example 2 further derivatives may be produced in comparable yields.

Starting the reaction sequence with *R*- or *S*- ethyl-2-hydroxy-4-methyl-2-(trifluoromethyl)pentenoate in combination with the compounds of general formula IV as described above the following compounds according to formula I may be produced in enantiomerically pure form:

| | **Z¹** | **Z²** | **Z³** | **X¹** | **X²** | **X³** | **enantiomer** |
|---|---|---|---|---|---|---|---|
| a) | O | | | F | | | *R* |
| b) | | O | | | F | | *R* |
| c) | | | O | | | F | *R* |
| d) | NH | | | F | | | *R* |
| e) | | | O | | F | | *R* |
| f) | S | | | | | F | *R* |
| g) | | NH | | Cl | | | *R* |
| h) | | | NH | | Cl | | *R* |
| i) | | | S | | | Cl | *R* |
| j) | | S | | CF₃ | | | *R* |
| k) | S | | | | CF₃ | | *R* |
| l) | O | | | | | CF₃ | *R* |
| m) | | O | | O-CH₃ | | | *R* |
| n) | | | O | | O-CH₃ | | *R* |
| o) | | O | O | | | O-CH₃ | *R* |
| p) | O | | | | F | | *R* |
| q) | NH | | | | | F | *R* |
| r) | | NH | | NH₂ | | | *R* |
| s) | | | NH | | NH₂ | | *R* |
| t) | | | O | | | Br | *R* |
| u) | O | | | F | | | *S* |
| v) | | O | | | F | | *S* |
| w) | | | O | | | F | *S* |
| x) | NH | | | F | | | *S* |
| y) | | | O | | F | | *S* |
| z) | S | | | | | F | *S* |
| aa) | | NH | | Cl | | | *S* |
| bb) | | | NH | | Cl | | *S* |
| cc) | | | S | | | Cl | *S* |
| dd) | | S | | CF₃ | | | *S* |
| ee) | S | | | | CF₃ | | *S* |
| ff) | O | | | | | CF₃ | *S* |
| gg) | | O | | O-CH₃ | | | *S* |
| hh) | | | O | | O-CH₃ | | *S* |
| ii) | | O | O | | | O-CH₃ | *S* |
| jj) | O | | | | F | | *S* |
| kk) | NH | | | | | F | *S* |
| ll) | | NH | | NH₂ | | | *S* |
| mm) | | | NH | | NH₂ | | *S* |
| nn) | | | O | | | Br | *S* |

### 4) Synthesis of 4-(fluoro-2,3-dihydrobenzo-furan-7-yl)-4-methyl-2-(trifluoromethyl)pentanal]

To a solution of 7,5 mmol [4-(5-fluoro-2,3-dihydrobenzo-furan-7-yl)-4-methyl-2-(trifluoromethyl)pentane-1,2-diol] in 79 ml of dichloromethane, 26 ml of dimethylsulfoxide (DMSO), and 5,2 ml of triethylamine are added at 25°C and stirred for 20 minutes. 2,7 mmol of SO₃/pyridine complex are added portionwise over 30 minutes. The mixture is further stirred at this temperature for at least 3 hours. After complete reaction (TLC control) the mixture is treated with 46 ml of saturated amoniumchloride solution and stirred for 40 minutes. Finally, the mixture is extracted four times with 56 ml of MTBE. The combined organic phases are washed with 26 ml of brine. The solvent is evaporated under vacuum. The residue obtained is purified by filtration over a small pad of silica gel (yield 87,9%).

### 5) Synthesis of 1,1,1 trifluoro-4-(5-fluoro-2,3-dihydrobenzofuran-7-yl)-4-methyl-2-{[(2-methyl-5-quinoline-5-ylimino]methyl}pentane-2-ol)

To a solution of 3,84 g 4-(fluoro-2,3-dihydrobenzo-furan-7-yl)-4-methyl-2-(trifluoromethyl)pentanal] in 7 ml of acetic acid is added 2,28 g of 5-amino-2-methylquinoline at 25°C. The mixture is stirred at 25°C for 16 hours. 50 ml of toluene is added to the solution and refluxed under Dean-Stark trap for at least 12 hours. After complete reaction (TLC control) the solvent is evaporated under vacuum. Acetic acid is removed by aceotropic destillation with toluene. The residue is purified by filtration over a small pad of silica gel. (Yield 88,7 %).

The reaction may be carried out under similar conditions with the amines listed below with comparable results:
5-amino-2-methylquinoline
5-amino-quinoline
3-amino-quinoline
5-amino-1-methyl-isoquinoline
5- amino-isoquinoline
5-amino-isochinol-2(1H)-one
5-amino-2,6-di-methylquinoline
5-amino-6-chloro-2-methylquinoline
5-amino-6-fluoro-2-methylquinoline
5-amino-2-methyl-isochinol-1 (2H)-one
amino-benzene
1-amino-2-methyl-benzene
1-amino-4-methyl-benzene
2-amino-pyrimidine
4-amino-pyrimidine
2-amino-pyridine
4-amino-pyridine
2-amino-4-methylpyridine
N-(4-aminophenyl)-piperidine
5-amino-2-methyl-indole

### 6)Synthesis of 1,1,1 trifluoro-4-(5-fluoro-2,3-dihydrobenzofuran-7-yl)-4-methyl-2-{[(2-methyl-5-quinolinyl]methyl}pentane-2-ol)

10 mmol 1,1,1 trifluoro-4-(5-fluoro-2,3-dihydrobenzofuran-7-yl)-4-methyl-2-{[(2-methyl-5-quinoline-5-ylimino]methyl}pentane-2-ol) are dissolved in 255 ml of methanol. To this solution 5 mmol of sodium bicarbonate is added. The mixture is stirred at 25 °C for 20 minutes. 34 mmol of sodium boronhydride are added to this solution during 10 minutes maintaining the temperature at 25°C. The mixture is stirred for 6 hours and another 34 mmol portion of sodium boronhydride is added to the solution over 10 minutes maintaining the temperature at 25°C. Then the mixture is stirred at room temperature for 6 hours (TLC control). After completion saturated sodium bicarbonate solution is added over 10 minutes keeping the temperature at 25°C. The mixture is stirred for 60 minutes, and finally the solvent is evaporated under vavuum. The residue is diluted with water and extracted two times with 150 ml of ethyl acetate each. The solvent is evaporated and the residue obtained is purified by recrystalization from methanol (yield 71,2 %).

Using other amines in the reaction of example 5 (e.g. those listen in example 5) and using the other compounds of formula I (e.g. those listed in the table in example 3) quite a number of compounds of general formula VIII may be generated using the methods described herein.

According to the examples described above, it is possible to synthesize the compound according to general formula VIII in 6 steps, whereas the prior art methods needed 13 steps. The overall yield of the present 6 steps synthesis of compounds of general formula VIII is 14,3% whereas it is 8,7% using the prior art methods.
Moreover, the whole synthetic route can be carried out in enantiomerically pure form, i.e. generating only the wanted enantiomer of general formula VIII. This is possible in using chiral alkyl 2-hydroxy-4-methyl-2-(trifluoromethyl)pentenoate.
It is important to understand that the total yield using chiral alkyl 2-hydroxy-4-methyl-2-(trifluoromethyl)pentenoate remains approximately 14 % whereas it drops to less than 5 % according to prior art methods due to the necessary separation of the enantiomers.
The reaction conditions according to the described steps are moreover suitable for production in industrial scale.

## Claims

1. A process for the manufacturing of a compound according to general formula I 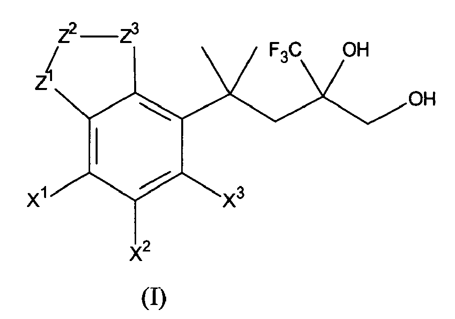 in which one of the groups X¹, X², X³ is selected from
fluoro, chloro, bromo, hydroxy, methoxy, ethoxy, trifluoromethyl, amino
whereas the other groups X¹, X², X³ have the meaning of a hydrogen atom,
and in which one of the groups Z¹, Z², Z³ is selected from
-O-, -S-, -NH-, -N(-CH₃)-,
whereas the other groups Z¹, Z², Z³ have the meaning of a -CH₂- group
**characterized in that** a compound according to general formula IV 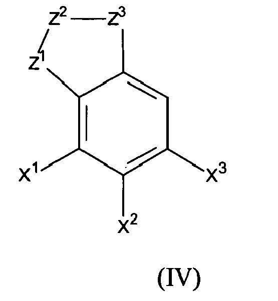 in which X¹, X², X¹, Z¹, Z², Z³ have the above described meaning
is reacted with alkyl 2-hydroxy-4-methyl-2-(trifluormethyl)pentenoate to yield a compound of general formula III 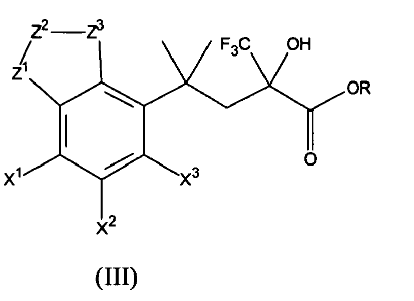 in which X¹, X², X³, Z¹, Z², Z³ have the above described meaning and in which R is a C₁-C₅-alkyl group, which might be straight or branched
which is then saponified to a compound of general formula II 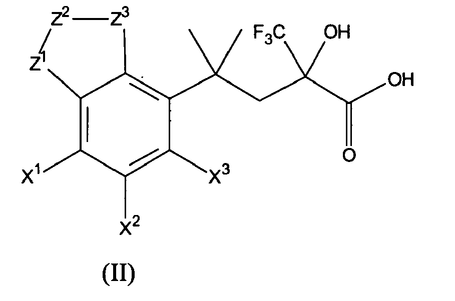 in which X¹, X², X¹, Z¹, Z², Z³ have the above described meaning
and subsequently reduced to a compound of general formula I.

2. A process according to claim 1 in which the reaction of the compound according to formula IV with alkyl 2-hydroxy-4-methyl-2-(trifluormethyl)pentenoate is carried out in a polar solvent in the presence of AlCl₃.

3. A process according to claim 2 in which the compound according to formula IV is solved in a halogenated solvent, AlCl₃ is added and finally the alkyl 2-hydroxy-4-methyl-2-(trifluormethyl)pentenoate is added to the stirred solution.

4. A process according to claim 3 in which the compound according to formula IV is solved in dichloromethane, AlCl₃ is added at 0-5°C and the alkyl 2-hydroxy-4-methyl-2-(trifluormethyl)pentenoate is added to the stirred solution and continued to stir for 1-5 days at room temperature.

5. A process according to claim 4 in which approximately 1.5 Eq. of the compound according to formula IV is solved in dichloromethane, approximately 2 Eq.AlCl₃ is added at 0-5°C and approximately 1.0 Eq. of the alkyl 2-hydroxy-4-methyl-2-(trifluormethyl)pentenoate is added to the stirred solution and continued to stir for 1-5 days at room temperature.

6. A process according to claim 1 in which the alkyl 2-hydroxy-4-methyl-2-(trifluormethyl)pentenoate is used in enantiomeric pure form to yield enantiomeric pure compound of formula I.

7. A process according to claim 1 in which the enantiomeric pure alkyl 2-hydroxy-4-methyl-2-(trifluormethyl)pentenoate is generated by enzymatic hydrolysis.

8. A process according to claim 7 in which the enzymatic hydrolysis is carried out in a buffered DMSO solution.

9. A process according to claims 7 or 8 in which the enzyme is selected from:
Lipase AH, Lipase AK, Lipase AYS, Lipase PS, Protease K, Protease N, Protease P
Lipase C1, Lipase C2, Lipase A, Lipase AS1, Lipase AN, Lipase PC, Lipase PF, Lipase B (CALB)
ICR-101, ICR-102, ICR-103, ICR-104, ICR-105, ICR-106, ICR-107, ICR-108, ICR-109,
ICR-110, ICR-111, ICR-112, ICR-113, ICR-114, ICR-115, ICR-116, ICR-117, ICR-118,
ICR-119, ICR-120, IMW-102, IMW-105
Esterase BS1, Esterase BS2, Esterase BS3, Esterase PF2, Esterase PL
Duramyl, Novozyme 868, Novozyme 525L, Novozyme 388, Neutrase 0, Liopoase
Lipase from porcine pancreas Typ II, Esterase porcine liver, Lipase candida rugosa.

10. A process according to at least one of claims 1-9 in which the compound of formula IV has the following substitution pattern:
| | Z¹ | Z² | Z³ | X¹ | X² | X³ |
|---|---|---|---|---|---|---|
| a | O | | | F | | |
| b | | O | | | F | |
| c | | | O | | | F |
| d | NH | | | F | | |
| e | | | O | | F | |
| f | S | | | | | F |
| g | | NH | | Cl | | |
| h | | | NH | | Cl | |
| i | | | S | | | Cl |
| j | | S | | CF₃ | | |
| k | S | | | | CF₃ | |
| l | O | | | | | CF₃ |
| m | | O | | O-CH₃ | | |
| n | | | O | | O-CH₃ | |
| o | | O | O | | | O-CH₃ |
| p | O | | | | F | |
| q | NH | | | | | F |
| r | | NH | | NH₂ | | |
| s | | | NH | | NH₂ | |
| t | | | O | | | Br |

11. A compound of formula I which has the following substitution pattern:
| | **Z¹** | **Z²** | **Z³** | **X¹** | **X²** | **X³** | **enantiomer** |
|---|---|---|---|---|---|---|---|
| a) | O | | | F | | | *R* |
| b) | | O | | | F | | *R* |
| c) | | | O | | | F | *R* |
| d) | NH | | | F | | | *R* |
| e) | | | O | | F | | *R* |
| f) | S | | | | | F | *R* |
| g) | | NH | | Cl | | | *R* |
| h) | | | NH | | Cl | | *R* |
| i) | | | S | | | Cl | *R* |
| j) | | S | | CF₃ | | | *R* |
| k) | S | | | | CF₃ | | *R* |
| l) | O | | | | | CF₃ | *R* |
| m) | | O | | O-CH₃ | | | *R* |
| n) | | | O | | O-CH₃ | | *R* |
| o) | | O | O | | | O-CH₃ | *R* |
| p) | O | | | | F | | *R* |
| q) | NH | | | | | F | *R* |
| r) | | NH | | NH₂ | | | *R* |
| s) | | | NH | | NH₂ | | *R* |
| t) | | | O | | | Br | *R* |
| u) | O | | | F | | | *S* |
| v) | | O | | | F | | *S* |
| w) | | | O | | | F | *S* |
| x) | NH | | | F | | | *S* |
| y) | | | O | | F | | *S* |
| z) | S | | | | | F | *S* |
| aa) | | NH | | Cl | | | *S* |
| bb) | | | NH | | Cl | | *S* |
| cc) | | | S | | | Cl | *S* |
| dd) | | S | | CF₃ | | | *S* |
| ee) | S | | | | CF₃ | | *S* |
| ff) | O | | | | | CF₃ | *S* |
| gg) | | O | | O-CH₃ | | | *S* |
| hh) | | | O | | O-CH₃ O-CH₃ | | *S* |
| ii) | | O | O | | | O-CH₃ | *S* |
| jj) | O | | | | F | | *S* |
| kk) | NH | | | | | F | *S* |
| ll) | | NH | | NH₂ | | | *S* |
| mm) | | | NH | | NH₂ | | *S* |
| nn) | | | O | | | Br | *S* |

12. A compound of claim 11 **characterized by** an enantiomeric excess (ee) >80%.

13. A process for the manufacturing of a compound according to general formula VIII 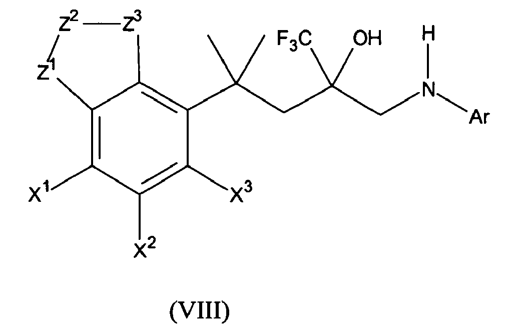 in which one of the groups X¹, X², X³ is selected from
fluoro, chloro, bromo, hydroxy, methoxy, ethoxy, trifluoromethyl, amino
whereas the other groups X¹, X², X³ have the meaning of a hydrogen atom,
and in which one of the groups Z¹, Z², Z³ is selected from
-O-, -S-, -NH-, -N(-CH₃)-,
whereas the other groups Z¹, Z², Z³ have the meaning of a -CH₂- group and in which Ar stands for an aromatic group,
**characterized in that** a compound according to general formula I 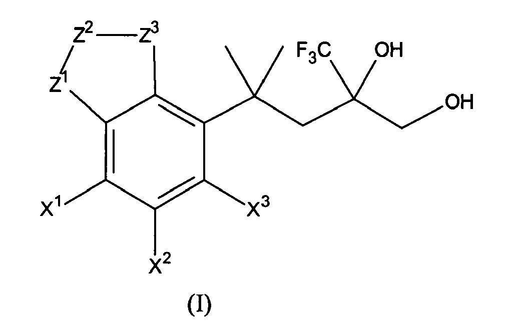 in which X¹, X², X³, Z¹, Z², Z³ have the above described meaning
is oxidized to form the aldehyde of formula V 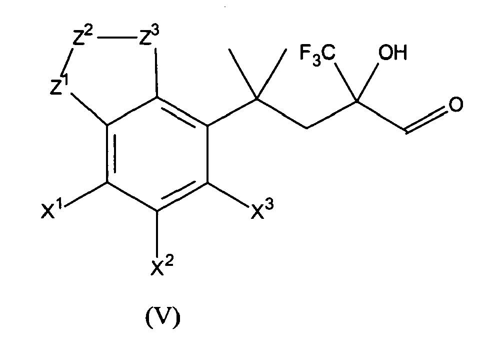 in which X¹, X², X³, Z¹, Z², Z³ have the above described meaning
which is then reacted with an aromatic amine of formula VI
H₂N-Ar (VI)
in which Ar is an aromatic group
to an imine of formula VII 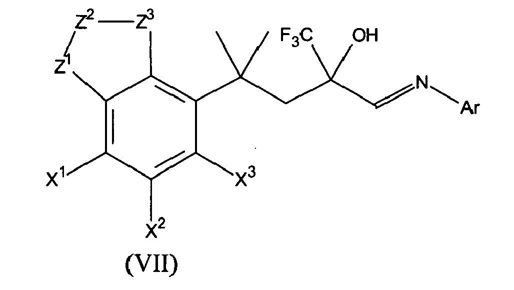 in which X¹, X², X³, Z¹, Z², Z³ and Ar have the above described meaning
which is subsequently reduced to form the compound according to formula VIII.

14. A process according to claim 13 in which the compound of general formula I is reacted with SO₃/pyridine complex to form the aldehyde of formula V.

15. A process according to claim 13 in which the compound of general formula V is solved in acetic acid, the amine of formula VI is added at room temperature and stirred for 5-30 h, toluene is added and the mixture is refluxed for 5-50 h to yield the imine of formula VII.

16. A process according to claim 13 or 15 in which the amine of formula VI is selected from:
5-amino-2-methylquinoline
5-amino-quinoline
3-amino-quinoline
5-amino-1-methyl-isoquinoline
5- amino-isoquinoline
5-amino-isochinol-2(1H)-one
5-amino-2,6-di-methylquinoline
5-amino-6-chloro-2-methylquinoline
5-amino-6-fluoro-2-methylquinoline
5-amino-2-methyl-isochinol-1 (2H)-one
amino-benzene
1-amino-2-methyl-benzene
1-amino-4-methyl-benzene
2-amino-pyrimidine
4-amino-pyrimidine
2-amino-pyridine
4-amino-pyridine
2-amino-4-methylpyridine
N-(4-aminophenyl)-piperidine
5-amino-2-methyl-indole

17. A process according to claim 16 in which the imine of formula VII is reacted with sodium borohydride in alcoholic solution to yield the compound according to formula VIII.

18. A process according to claim 13 in which the compound of formula I is used in an enantiomeric pure form to yield an enantiomeric pure compound of formula VIII.
